# EUROPEAN PATENT APPLICATION

(11) **EP 3 800 246 A1**
(43) Date of publication of application: **07.04.2021**
(21) Application number: 19872026.0
(22) Date of filing: 10.10.2019
(51) Int. Cl.: C12N 5/10, C12N 15/07

(54) **METHOD FOR PRODUCING HUMAN INDUCED PLURIPOTENT STEM CELLS CONTAINING EXOGENOUS CHROMOSOME**

(30) Priority: 10.10.2018 JP 2018191894
(71) Applicant: National University Corporation Tottori University, Tottori-shi, Tottori 680-8550 (JP); Trans Chromosomics, Inc., Yonago-shi, Tottori 683-8503 (JP)
(72) Inventor: KAZUKI Yasuhiro, Yonago-shi, Tottori 683-8503 (JP); UNO Narumi, Yonago-shi, Tottori 683-8503 (JP); OSHIMURA Mitsuo, Yonago-shi, Tottori 683-8503 (JP)
(74) Representative: D Young & Co LLP
(86) International application number: PCT/JP2019/040090
(87) International publication number: WO 2020/075822

(57) **Abstract**

The present application provides: a method for producing human induced pluripotent stem (iPS) cells comprising an exogenous chromosome having a DNA of interest using the MMCT method; and a method for expressing the exogenous gene in the human iPS cells prepared by the method for producing human iPS cells, or in undifferentiated or differentiated cells derived from the human iPS cells induced to differentiate from the human iPS cells.

## Description

### TECHNICAL FIELD

The present invention relates to a method for producing human induced pluripotent stem cells comprising an exogenous chromosome.

Specifically, the present invention relates to a method for producing human induced pluripotent stem cells comprising an exogenous chromosome, characterized by use of MMCT (Microcell-Mediated Chromosome Transfer) method using a viral envelope protein.

Examples of the exogenous chromosome include artificial chromosomes, such as mammalian artificial chromosomes, human artificial chromosomes, and mouse artificial chromosomes.

The present invention also relates to a method for expressing at least one exogenous gene in an exogenous chromosome in the above-mentioned human induced pluripotent stem cells, or in undifferentiated or differentiated cells derived from the human induced pluripotent stem cells.

### BACKGROUND ART

MV-MMCT (Microcell-Mediated Chromosome Transfer using Measles virus fusogen) method is a technique developed by the present inventors and the use thereof enables the transfer of chromosomes by the microcell fusion method from donor cells to recipient cells. Specifically, when genes encoding hemagglutinin (H) protein and fusion (F) protein derived from an attenuated measles virus (MV) are transfected into donor cells carrying an exogenous chromosome, an MV fusion envelope protein is expressed on the surface of the donor cells. These cells are further treated with colcemid to form microcells, which cause microcell fusion when co-cultured with recipient cells whereby the recipient cells into which the target exogenous chromosome has been transferred are obtained by a selection method such as drug selection (Non Patent Literatures 1 to 3).

Also developed is an MMCT method (retro-MMCT) by which an envelope protein derived from, for example, a leukemia virus in place of the above MV is expressed on the surface of donor cells (Non Patent Literature 4). Specifically, this literature describes a highly efficient transfer method ("retro-MMCT method" (retro-MMCT)) of introducing a chromosome (for example, human artificial chromosomes or mouse artificial chromosomes) into various target cells using Chinese hamster ovarian (CHO) cells that express envelope proteins derived from mouse leukemia virus (composed of "the SU component" and "the TM component").

Gene transfer vectors are used distinguishably depending on the gene size capable of carrying on the vectors. For example, polynucleotides having a size of about 20 kb or less are for a plasmid, polynucleotides having a size of 150 kb or less are for a virus vector, polynucleotides having a size of 300 kb or less are for a BAC/PAC, and polynucleotides having a size of about less than 1 Mb are for a YAC, while artificial chromosomes such as human artificial chromosomes and mouse artificial chromosomes have no limitation in the chromosome size to be transferred (Non Patent Literature 5). For this reason, these artificial chromosomes can carry a chromosome fragment comprising a gene locus of interest.

The above-mentioned human artificial chromosomes and mouse artificial chromosomes were developed for the first time by the present inventors and their names are different depending on the origin of a chromosome. For example, the artificial chromosome derived from a human chromosome is called a human artificial chromosome, while the artificial chromosome derived from a mouse chromosome is called a mouse artificial chromosome (Patent Literatures 1, 2, and 3).

Induced pluripotent stem (iPS) cell is a stem cell having characteristics similar to an embryonic stem (ES) cell artificially prepared from somatic cells by Dr. Shinya Yamanaka (Kyoto University) and has infinite proliferation and pluripotency (Non Patent Literatures 6 and 7). An iPS cell can be differentiated into somatic cells (stem cells, progenitor cells, or mature cells) of various tissues and thus the application to regenerative medicine has been promoted, and further because iPS cells induced from somatic cells of a patient with a genetic disease form disease model cells, they have been utilized for pharmaceutical development (Non Patent Literature 8).

### CITATION LIST

### PATENT LITERATURE

Patent Literature 1: JP Patent No. 4997544
Patent Literature 2: JP Patent No. 5557217
Patent Literature 3: JP Patent No. 4895100

### NON PATENT LITERATURE

Non Patent Literature 1: M. Katoh et al., BMC Biotechnology 2010, 10:37
Non Patent Literature 2: N. Uno et al., Cytotechnology 2013, 65:803-809
Non Patent Literature 3: M Hiratsuka et al., BMC Biotechnology 2015, 15:58
Non Patent Literature 4: T. Suzuki et al., PLOS ONE, DOI:10.1371/journal.pone.0157187 June 7, 2016
Non Patent Literature 5: P. Osten et al., Handb Exp Pharmacol 2007, 178:177-202
Non Patent Literature 6: K. Takahashi and S. Yamanaka, Cell 2006, 126(4):663-676
Non Patent Literature 7: K. Takahashi et al., Cell 2007, 131(5):861-872
Non Patent Literature 8: V.K. Singh et al., Frontiers in Cell and Developmental Biology 2015, doi:10.3389/fcell.2015.00002

### SUMMARY OF THE INVENTION

### Problem to be Solved by the Invention

For example, the development of techniques for introducing an exogenous chromosome comprising a DNA of interest (e.g., a gene, a gene locus, or a chromosome fragment) into human induced pluripotent stem cells (hereinafter, also referred to as "iPS cells") is important for regenerative medicine or pharmaceutical development, but the gene transfer by the conventional transgenic technique has the limitation in the introducible gene size, and because a transferred gene is integrated in a chromosome of a host, there were problems of safety and gene expression control.

Use of the conventional gene transfer methods (e.g., plasmids, BAC, PAC, and YAC) failed to introduce a giant DNA (e.g., a chromosome, a fragment thereof, and an artificial chromosome) having a megabase (Mb) size at once into human iPS cells. Additionally, when a chromosome was transferred into human iPS cells, a culture method on feeder cells was used but failed to transfer the chromosome into the human iPS cells. Further, when a chromosome was transferred into human iPS cells, the MMCT method using a polyethyleneglycol (PEG) method was used but failed to transfer the chromosome into the human iPS cells.

An object of the present invention is to provide a method for producing human iPS cells comprising an exogenous chromosome having a megabase (Mb) size. In this method, when the exogenous chromosome is an artificial chromosome (e.g., mammalian artificial chromosome human artificial chromosome, or mouse artificial chromosome), the artificial chromosome is not integrated in the genome of human iPS cells.

### Means for Solution of the Problem

The present invention encompasses the following features.
(1) A method for producing human induced pluripotent stem (iPS) cells comprising an exogenous chromosome, comprising the following steps of:
   providing a donor cell expressing a viral envelope protein on the cell surface and comprising an exogenous chromosome having a DNA of interest;
   preparing microcells from the donor cell; and
   co-culturing and fusing the microcells and human iPS cells as a recipient cell in the absence of feeder cells using an MMCT (Microcell-Mediated Chromosome Transfer) method, thereby introducing the exogenous chromosome having a DNA of interest into the human iPS cells.
(2) The method according to (1) above, wherein the exogenous chromosome is an artificial chromosome or a mammalian artificial chromosome.
(3) The method according to (1) or (2) above, wherein the viral envelope protein is a measles virus-derived envelope protein or a modified protein thereof.
(4) The method according to (3) above, wherein the modified protein is a fusion protein of a measles virus-derived H protein with an anti-CD9 antibody, anti-CD 13 antibody or anti-CD71 antibody, or with an ScFv of the antibody.
(5) The method according to any of (1) to (4) above, wherein the donor cell is a mammalian cell.
(6) The method according to (5) above, wherein the mammalian cell is a rodent cell.
(7) The method according to any of (1) to (6) above, wherein the DNA of interest is an exogenous gene, a locus, or a chromosome fragment.
(8) A method for expressing an exogenous gene in human iPS cells or in undifferentiated or differentiated cells derived from the human iPS cells, the method comprising the following steps of:
   preparing human iPS cells comprising an exogenous chromosome having an exogenous gene by the method according to any one of claims 1 to 7; and
   expressing the exogenous gene in the human iPS cells or in the undifferentiated or differentiated cells derived from the human iPS cells and induced to differentiate from the human iPS cells.
(9) The method according to (8) above, wherein the undifferentiated or differentiated cell derived from the human iPS cells is a stem cell, a hematopoietic stem cell, a mesenchymal stem cell, a muscle satellite cell, a progenitor cell, a mature cell, or a cell population thereof.
(10) The method according to (9) above, wherein the undifferentiated or differentiated cell derived from the human iPS cells is selected from the group consisting of nerve cells, myocardial cells, skeletal muscle cells, smooth muscle cells, T cells, B cells, NK cells, megakaryocytes, hepatocytes, epithelial cells, endothelial cells, pancreatic cells, nephrocytes, and small intestinal cells.
(11) The method according to any of (8) to (10) above, comprising an insulator-like DNA sequence upstream and/or downstream of a DNA comprising the exogenous gene.

The present description encompasses the disclosed contents of JP Patent Application No. 2018-191894 from which the present application claims priority.

The present invention enables efficient transfer of an exogenous chromosome into human iPS cells thereby to provide usefulness of using human iPS cells, when the exogenous chromosome comprises a DNA of interest, for regenerative medicine and pharmaceutical development.

### BRIEF DESCRIPTION OF THE DRAWINGS

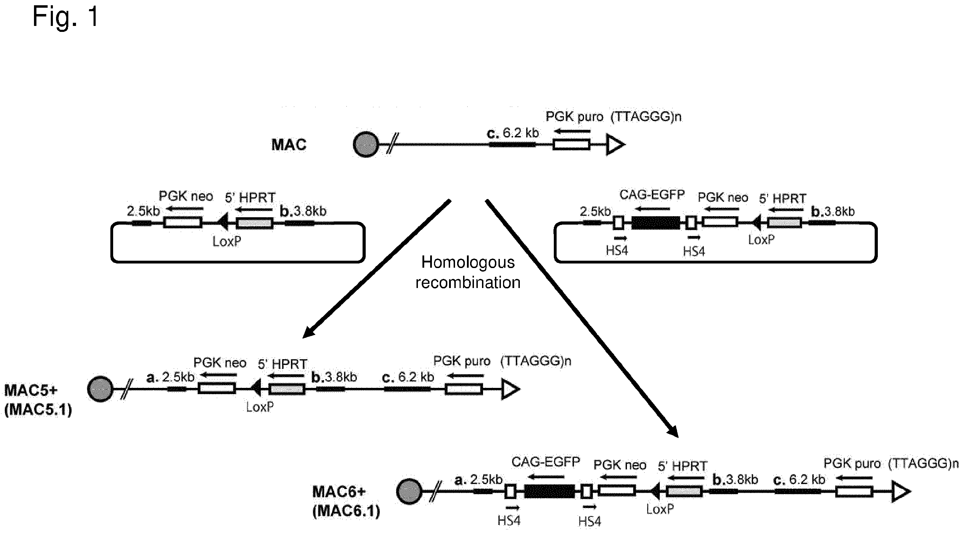

[Fig. 1] This figure shows the preparation procedure and structures of MAC5 and MAC6 by the method of Takiguchi et al. (ACS Synth. Biol. 2014; 3:903-914). In the figure, black circle represents a centromere, and white triangle represents a telomere. Additionally, HS4 represents an insulator, CAG and PGK each represent a promoter, EGFP represents a fluorescent protein-encoding gene, neo and puro each represent a drug resistant gene, and HPRT represents a hypoxanthine-guanine phosphoribosyl transferase gene.

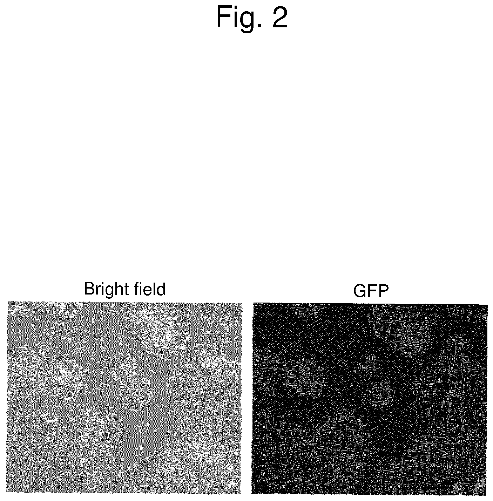

[Fig. 2] This figure shows fluorescence-microscopic images where a MAC vector has been transferred into a human iPS cell line. The left panel shows a bright field image and the right panel shows a corresponding GFP fluorescent field image.

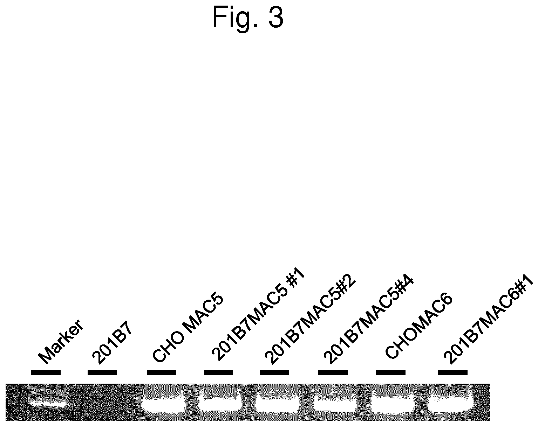

[Fig. 3] This figure shows the results of PCR analyses of human iPS cell clones into which MAC vectors (MAC5, MAC6) have been transferred. 201B7 is a human iPS cell into which no vector is transferred and is a negative control with no band indication. CHO MAC5 and CHO MAC6 are positive controls which carry the MAC vector. 201B7MAC5#1, 201B7MAC5#2, 201B7MAC5#4, and 201B7MAC6#1 are clones carrying the transferred MAC vector.

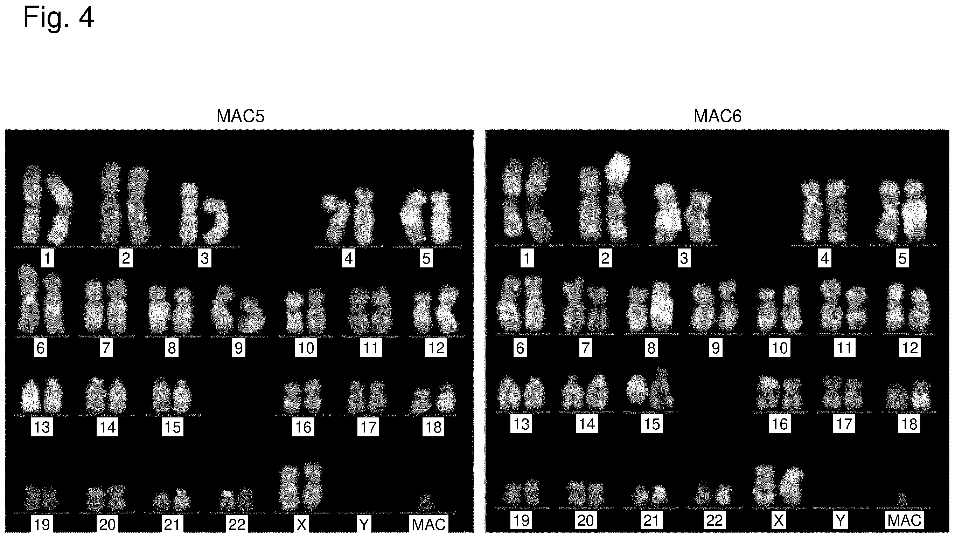

[Fig. 4] This figure shows the results of karyotype analyses using quinacrine-Hoechst staining of the clones in which MAC5 (left panel) or MAC6 (right panel) has been transferred into the human iPS cell line 201B7. The chromosomes 1 to 22, X, Y and MAC are shown in the order in the direction of from the upper left to the lower right.

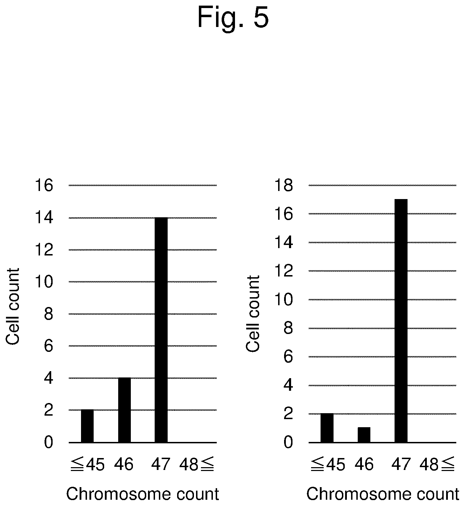

[Fig. 5] This figure shows the results of modal analyses using quinacrine-Hoechst staining of the clones in which MAC5 (left panel) or MAC6 (right panel) has been transferred into the human iPS cell line 201B7.

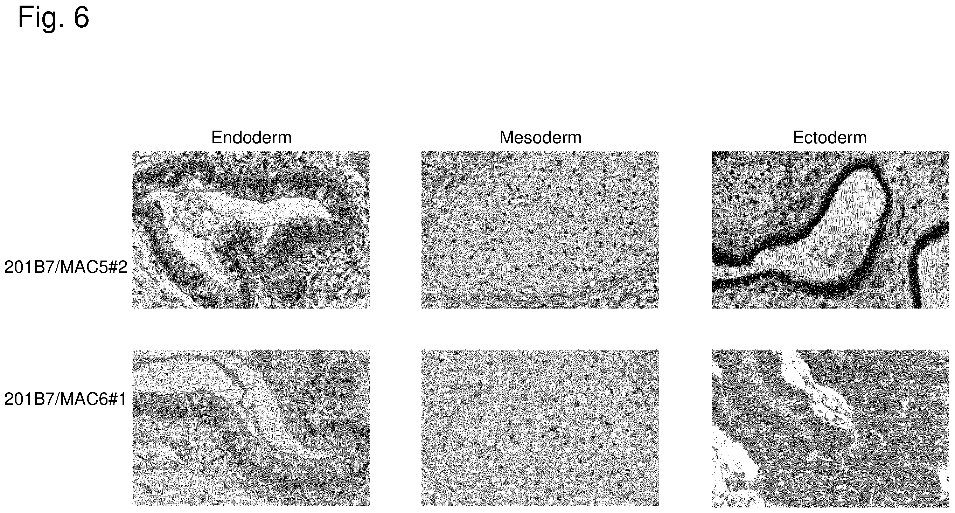

[Fig. 6] This figure shows hematoxylin and eosin-stained images of a teratoma derived from the human iPS cell line 201B7MAC5#2 (upper row) or 201B7MAC6#1 (lower row). Endodermal tissue (left panels), mesodermal tissue (center panels), and ectodermal tissue (right panels) observed are shown.

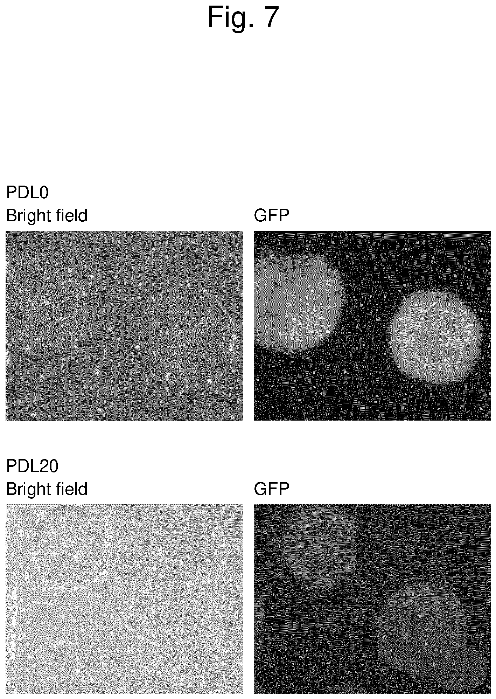

[Fig. 7] This figure shows GFP fluorescence images of the human iPS cell line 201B7MAC6#1 by fluorescence microscope. GFP fluorescence images at 0PDL before long-term culture (upper row) and GFP fluorescence images at 20PDL after long-term culture (lower row) are shown.

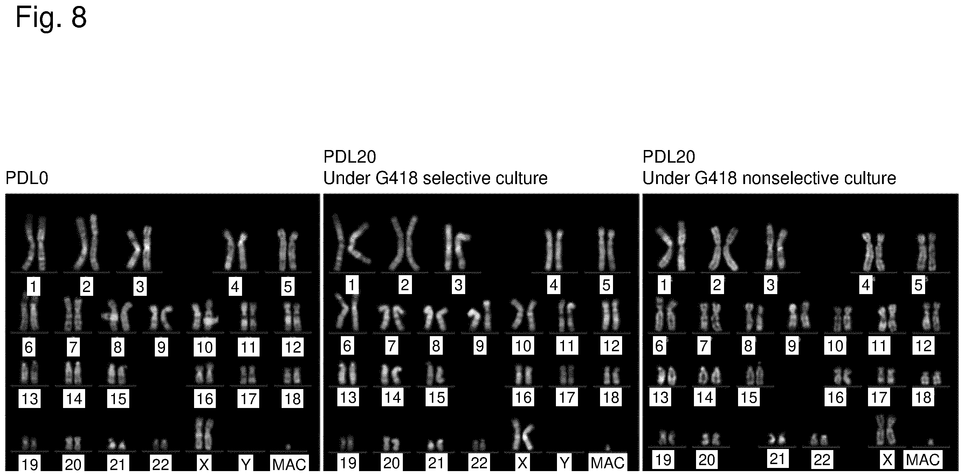

[Fig. 8] This figure shows the results of karyotype analyses using quinacrine-Hoechst staining of the long-term cultured human iPS cell line 201B7MAC6#1. The chromosomes 1 to 22, X, Y and MAC are shown in the order in the direction of from the upper left to the lower right. The analysis result at 0PDL before the long-term culture (left), the analysis result at 20PDL after the long-term culture under G418 selective culture (center), and the analysis result at 20PDL after the long-term culture under G418 nonselective culture (right) are shown.

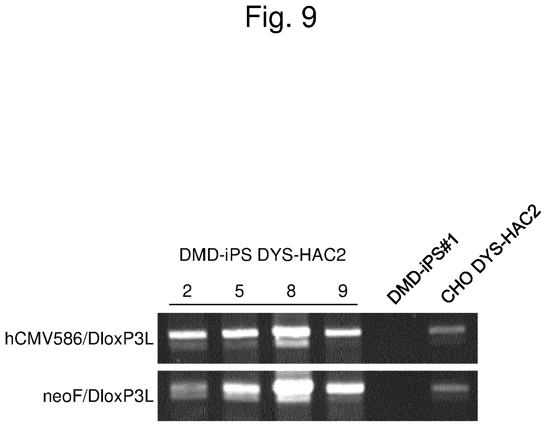

[Fig. 9] This figure shows the results of PCR analyses of clones in which DYS-HAC2 has been transferred into a human iPS cell line (DMD-iPS#1) when the primers indicated were used. DMD-iPS#1 is an iPS cell derived from a transfer-free DMD patient and a negative control in which no band of DYS-HAC2 appeared. CHO DYS-HAC2 is a positive control which carries the transferred DYS-HAC2 vector. DMD-iPS DYS-HAC2#2, 5, 8, and 9 are the clones which carry the transferred DYS-HAC2 vector.

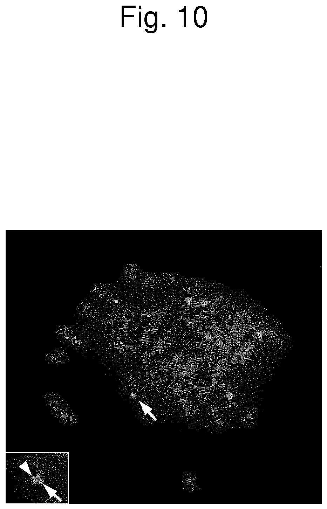

[Fig. 10] This figure shows a FISH staining result of a clone in which DYS-HAC2 has been transferred into the human iPS cell line (DMD-iPS#1) derived from a Duchenne muscular dystrophy (DMD) patient when human alpha satellite and RP11-954B16 were used as probes. Human No. 13, No. 21, or DYS-HAC2 is stained with red color (arrow). The dystrophin gene is stained with green color (arrowhead). The enlarged DYS-HAC2 vector is shown in a small window.

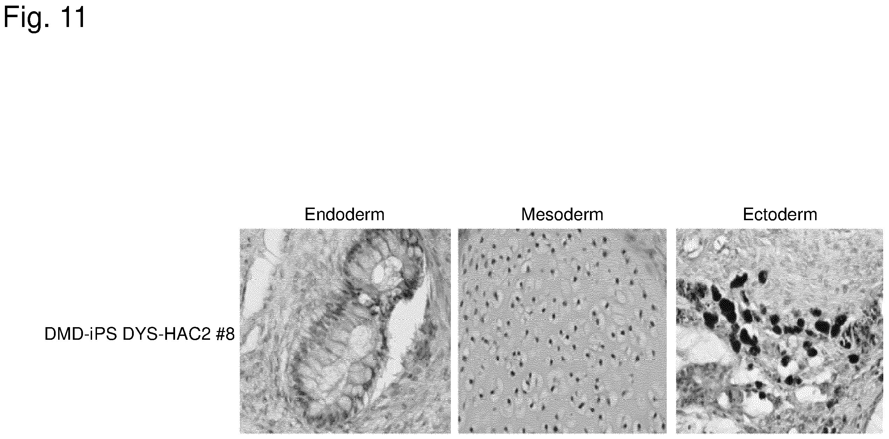

[Fig. 11] This figure shows hematoxylin and eosin-stained images of a teratoma derived from a clone DMD-iPS DYS-HAC2#8 in which DYS-HAC2 has transferred into a human iPS cell line derived from a DMD patient. Endodermal tissue (left panel), mesodermal tissue (center panel), and ectodermal tissue (right panel) observed are shown.

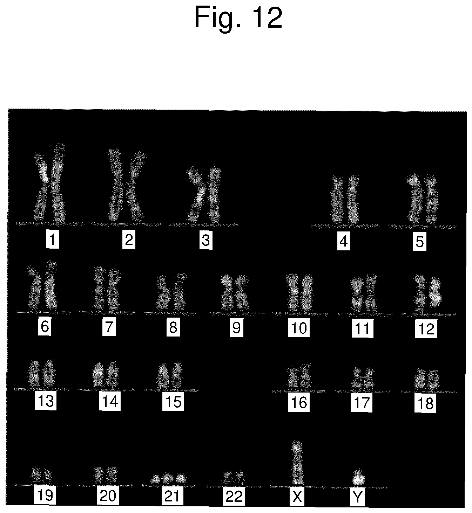

[Fig. 12] This figure shows the result of karyotype analysis with quinacrine-Hoechst staining of an exogenous human chromosome 21 fragment-comprising human iPS cell line in which the chromosome 21 has been transferred into a normal human iPS cell line. The chromosomes 1 to 22, X, and Y are shown in the order in the direction of from the upper left to the lower right. It is shown that a triplet of No. 21 chromosomes is contained.

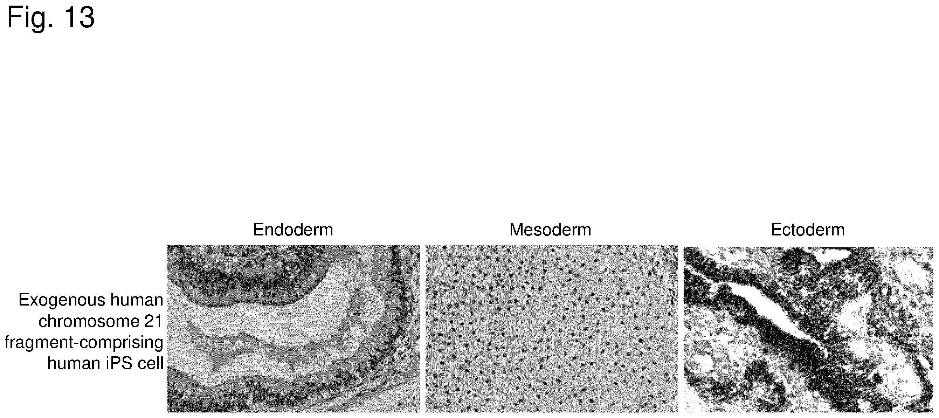

[Fig. 13] This figure shows hematoxylin and eosin-stained images of a teratoma derived from an exogenous human chromosome 21 fragment-comprising human iPS cell line in which the chromosome 21 is transferred into a normal human iPS cell line. Endodermal tissue (left panel), mesodermal tissue (center panel), and ectodermal tissue (right panel) observed are shown.

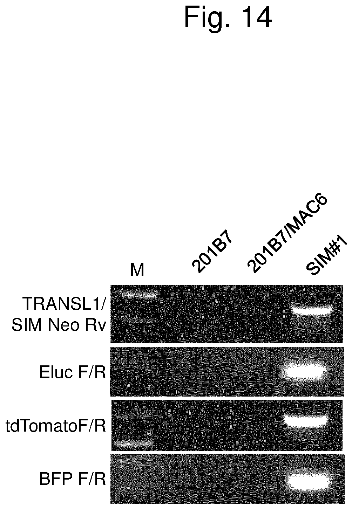

[Fig. 14] This figure shows the results of PCR analyses of human iPS cell clones in which an exogenous DNA was carried on a MAC vector when the primers indicated were used. 201B7 and 201B7/MAC6 are cells that did not have the exogenous DNA on a MAC vector and are negative controls in which no band of the exogenous DNA appeared. SIM#1 is a human iPS cell clone which carries the MAC vector comprising an exogenous DNA.

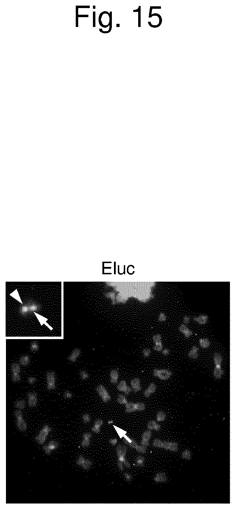

[Fig. 15] This figure shows a FISH staining result of a clone in which 3 types of plasmid vectors have been transferred on a MAC vector (MAC6) in a human iPS cell line (201B7/MAC6) when mouse Cot-1 DNA and pBG2-V0binsElucins were used as probes. The mouse artificial chromosome (MAC6) is stained with red color (arrow). The pBG2-V0binsElucins is stained with green color (arrowhead). The enlarged MAC6 vector into which the plasmid vector has been transferred is shown in a small window.

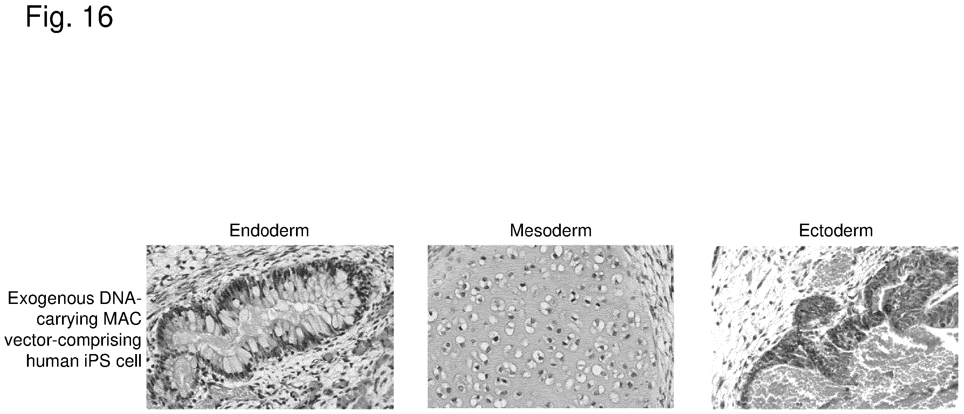

[Fig. 16] This figure shows hematoxylin and eosin-stained images of a teratoma derived from the human iPS cell clone SIM#1 that carries an exogenous DNA. Endodermal tissue (left panel), mesodermal tissue (center panel), and ectodermal tissue (right panel) observed are shown.

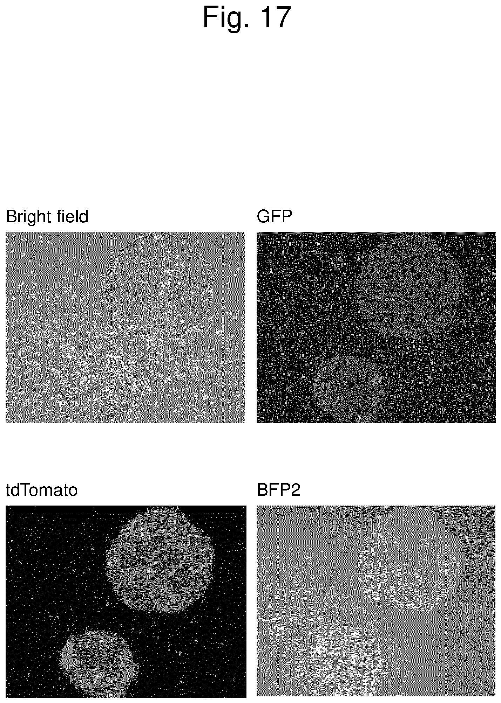

[Fig. 17] This figure shows GFP, tdTomato, and BFP2 fluorescence images, observed by fluorescence microscope, of the human iPS cell clone SIM#1 carrying an exogenous DNA. Each of the fluorescence images is shown at 0PDL before the long-term culture.

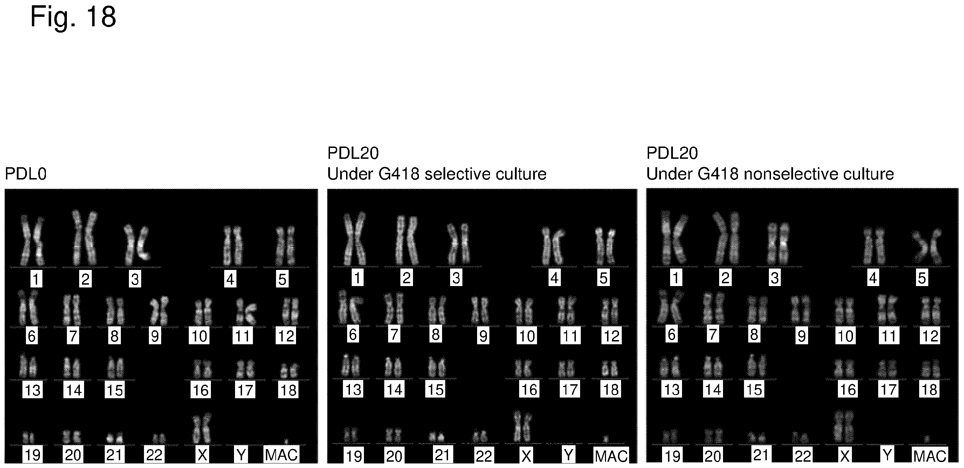

[Fig. 18] This figure shows the results of karyotype analyses using quinacrine-Hoechst staining of the human iPS cell clone SIM#1 carrying an exogenous DNA in the long-term culture. The chromosomes 1 to 22, X, Y and MAC are shown in the order in the direction of from the upper left to the lower right. The analysis result at 0PDL before the long-term culture (left panel), the analysis result at 20PDL after the long-term culture under G418 selective culture (center panel), and the analysis result at 20PDL after the long-term culture under G418 nonselective culture (right panel) are shown.

### Description of Embodiments

The present invention will be described in further detail.

### 1. Method for producing human iPS cells comprising an exogenous chromosome

The present invention provides a method for producing human iPS cells comprising an exogenous chromosome.

The above method comprises the following first step to third step to be described hereinafter.

### [First step]

This step is a step of providing donor cells expressing a viral envelope protein on the cell surface and comprising an exogenous chromosome having a DNA of interest or a DNA on interest.

The term "exogenous chromosome" as used herein refers to a chromosome that does not inherently exist in donor cells and includes, for example, chromosomes of mammalian animal cells that are of origins different from the donor cells, or chromosome fragments thereof, or artificially prepared chromosomes (referred to as "artificial chromosomes").

The term "artificial chromosome" as used herein refers to an artificially prepared chromosome-derived vector comprising the centromere of a chromosome derived from a mammalian animal (also referred to as "mammal") including human or rodent (for example, mouse, rat, or the like), any of fragments in the vicinity of the centromere of a long arm and, if any, a short arm (genes and the like are removed as much as possible), and natural or artificial telomere. Thus, the artificial chromosomes of the present invention are different from the conventional vector systems such as viruses, YAC, BAC, PAC, cosmids, and plasmids.

An exogenous chromosome comprises a site into which a DNA of interest (for example, a gene, a gene locus, or a chromosome fragment) is inserted, and the DNA of interest is inserted into this site. Examples of the exogenous chromosome include artificial chromosomes such as mammalian artificial chromosomes, mouse artificial chromosomes, and human artificial chromosomes.

Mouse artificial chromosomes are described in, for example, JP Patent No. 5,557,217 and JP Patent No. 4,997,544, and human artificial chromosomes are described in, for example, JP Patent No. 4,895,100.

A mouse chromosome used for preparing a mouse artificial chromosome may be any of the mouse chromosomes 1 to 19, X, and Y, preferably any of No. 1 to 19 chromosomes. For example, in the case of an artificial chromosome vector derived from a mouse chromosome 11 fragment, the long arm fragment is composed of, for example, but is not limited thereto, a long arm fragment from which a region distal to AL671968, or BX572640 (positioned at the more centromere side than AL671968), CR954170 (positioned at the more centromere side than AL671968 and BX572640), or AL713875 (positioned at the more centromere side than AL671968) of the long arm of the chromosome 11 is deleted. Alternatively, in the case of a mouse artificial chromosome derived from a mouse chromosome 15 fragment, the long arm fragment is composed of, for example, a long arm fragment from which a region distal to a position such as AC121307 or AC161799 is deleted. Alternatively, in the case of a mouse artificial chromosome derived from a mouse chromosome 16 fragment, the long arm fragment is composed of, for example, a long arm fragment from which a region distal to a position such as AC127687 or AC140982 is deleted.

A human chromosome used for preparing a human artificial chromosome may be any of the human chromosomes 1 to 22, X, and Y, preferably any of chromosomes 1 to 22. The preparation of a human artificial chromosome can refer to methods described in, for example, JP Patent Publication (Kokai) No. 2010-004887 A and WO2008/013067.

An exogenous chromosome may further comprise a DNA sequence insertion site such as loxP (Cre recombinase recognition site), FRT (Flp recombinase recognition site), φC31attB and φC31attP (φC31 recombinase recognition sites), R4attB and R4attP (R4 recombinase recognition sites), TP901-1attB and TP901-1attP (TP901-1 recombinase recognition sites), or Bxb1attB and Bxb1attP (Bxb1 recombinase recognition sites) for insertion of a DNA of interest (for example, a gene, a gene locus, or a chromosome fragment). Furthermore, as an exogenous chromosome may comprise a site for insertion of a DNA of interest sequence, the integration of a DNA of interest in this site enables the expression of the DNA of interest when such an exogenous chromosome is transferred into any cell.

Examples of the DNA of interest include, but are not limited to, useful genes, disease causative genes, therapeutic genes, chromosome fragments comprising these genes, genes required for cell differentiation, reporter genes, and selectable marker genes (positive selectable marker genes, negative selectable marker genes, etc.).

In the vicinity of an insertion site for a DNA of interest or on both sides of an insertion site in the exogenous chromosome of the present invention, at least one insulator sequence may be allowed to be present. The insulator sequence has an enhancer blocking effect (that is, neighboring genes are not affected by each other) or a chromosome boundary effect (that is, a region assuring the gene expression and a region suppressing the gene expression are separated and distinguished from each other). Such a sequence may include, for example, human β globins HS1 to HS5 or chicken β globin HS4.

For the donor cells, the DNA of interest described above is transferred into an exogenous chromosome by, for example, the following procedures. The gene transfer can be carried out by using, for example, a gene transfer method such as lipofection. At this time, the donor cells comprising the exogenous chromosome may be selected by a screening method such as a drug choice or HAT selection.

The donor cells are cells capable of forming microcells, preferably mammalian cells, and examples thereof include human cells and rodent cells. Specifically, the donor cells may include cells derived from tissues of mammalian internal organs or organs and, for example, cultured primary cells, established cell lines, immortalized cells, and ATCC deposited cells thereof. Examples of such cells include epithelial cells, fibroblast cells, endothelial cells, hepatocytes, muscle cells, chondrocytes, osteoblasts, myocardial cells, dermal papilla cells, nerve cells or neurons, ovarian cells, and iPS cells. Examples of the rodent cells include, but are not limited to, Chinese hamster ovarian (CHO) cells and mouse A9 cells.

The donor cell is further treated for expressing a viral envelope protein on the cell surface thereof.

The viral envelope protein includes envelope proteins derived from, for example, viruses such as measles virus, leukemia virus, ecotropic (retro)virus, amphotropic (retro)virus, and vesicular stomatitis virus, or modified proteins thereof. The envelope protein can comprise, in the case of the envelope protein derived from the measles virus, for example, hemagglutinin (H) protein and fusion (F) protein, and modified proteins thereof, and in the case of the envelope protein derived from the leukemia virus, for example, SU protein and TM protein, and modified proteins thereof. Preferable viral envelope proteins are envelope proteins derived from measles virus, such as H protein and F protein.

As a further example of the modified protein, H protein may be modified by the genetic engineering technique in such a way that the H protein can bind to a surface antigen of a specific recipient cell. For example, fusing an antigen-recognizing minimum unit of an antibody, i.e. single chain Fv (ScFv), preferably an anti-CD9 antibody, an anti-CD13 antibody or an anti-CD71 antibody, or ScFv of these antibodies, to the H protein enables the fusion to cells that cannot be fused by a wild-type H protein. Alternatively, a chromosome-receiving cell may be genetically modified so that CD46 antigen or CD120 (SLAM) antigen capable of binding to the H protein is expressed thereby to cause the fusion. Alternatively, a Tag peptide sequence may be added to the H protein, and a recipient cell may be genetically modified so that an anti-His tag antibody or an anti-H protein antibody is expressed thereby to cause the fusion. Alternatively, vesicular stomatitis virus G protein (VSV-G) is given as an example.

A plasmid comprising DNA encoding each of the above envelope proteins or a plasmid comprising a DNA cassette encoding a plurality of envelope proteins is constructed by the genetic recombination technology, and donor cells can be transformed using these plasmids.

For the selection of a target cell, drug resistant genes (for example, Blasticidin resistance gene and neomycin (G418) resistance gene) and/or genes encoding fluorescent proteins (for example, GFP and DsRed) can further be transferred into donor cells.

The above technique can prepare donor cells expressing a viral envelope protein on the cell surface and comprising an exogenous chromosome having a DNA of interest.

### [Second step]

This step is a step of preparing microcells from the donor cells of the first step.

For the preparation of microcells comprising an artificial chromosome from the donor cells, the donor cells are treated with a polyploid inducer (for example, colcemid or colchicine) thereby to form micro-multinucleated cells and further form microcells by cytochalasin treatment.

Treatment conditions for preparing microcells include culturing the donor cells for 72 hours under the conditions of, for example, 37°C and 5% CO₂ in Ham's F-12 medium containing 0.1 µg/mL of colcemid and 20% FCS.

### [Third step]

This step is a step of co-culturing and fusing the microcells of the second step and human iPS cells as recipient cells in the absence of feeder cells using the MMCT (Microcell-Mediated Chromosome Transfer; microcell fusion) method to introduce the exogenous chromosome comprising a DNA of interest into the human iPS cells.

The microcell fusion method used in the present invention is a method for transferring an artificial chromosome in the donor cells into recipient cells by the microcell fusion of microcells ("donor cells") derived from cells capable of forming microcells comprising an artificial chromosome, with other desired cells ("recipient cells").

According to the present invention, the use of a feeder-free culture method and the MMCT method using a viral envelope enables the transfer of an exogenous chromosome into human iPS cells.

iPS cells form colonies in about 3 to 5 weeks when certain reprogramming factors (DNAs or proteins) are transferred into somatic cells (including somatic stem cells) and then cultured and subcultured in a suitable medium. Known reprogramming factors include, for example, a combination consisting of Oct3/4, Sox2, Klf4, and c-Myc; a combination consisting of Oct3/4, Sox2, and Klf4; a combination consisting of Oct4, Sox2, Nanog, and Lin28; or a combination consisting of Oct3/4, Sox2, Klf4, c-Myc, Nanog, and Lin28 (K. Takahashi and S. Yamanaka, Cell; 126:663-676 (2006); WO2007/069666; M. Nakagawa et al., Nat. Biotechnol. 26:101-106 (2008); K. Takahashi et al., Cell; 131:861-872 (2007); J. Yu et al., Science; 318:1917-1920 (2007); J. Liao et al., Cell; Res. 18, 600-603 (2008)). Culture examples include culture in which a mitomycin C-treated mouse fetal fibroblast cell line (for example, STO) is used as the feeder cell and somatic cells (about 10⁴ to 10⁵ cells/cm²) into which a reprogramming factor expression vector has been transferred are cultured at a temperature of about 37°C on this feeder cell layer using medium for ES cells. At this time, the feeder cell is not always necessary (Takahashi, K. et al., Cell; 131:861-872 (2007)). Basal medium includes, for example, Dulbecco's Modified Eagle Medium (DMEM), Eagle's minimal essential medium (EMEM), Ham's F-12 medium, RPMI-1640 medium, Iscove's Modified Dulbecco's Medium (IMDM), and mixed media thereof, and primate ES cell medium (ReproCELL Incorporated, Japan) can be used as human iPS cell medium.

Examples of the culture medium for human iPS cells in the absence of feeder cells include StemFit™ AK02N (ReproCELL, Japan), TeSR™ (STEMCELL Technologies), and NutriStem XF/FF Culture Medium (STEM GWNT). Examples of the substrate for culture include Geltrrex™ LDEV-Free hESC-qualified (Thermo Fisher Scientific Inc.), iMatrix-511 (Nippi, Japan), and hES Qualified Matrigel™ (Corning).

The microcells prepared in the second step and human iPS cells are co-cultured in the medium described in the above examples in the absence of feeder cells to carry out the cell fusion. Subsequently, for example, culture is carried out in a medium containing a drug (for example, an antibiotic), and human iPS cells comprising an exogenous chromosome can be selected using the drug resistance (and fluorescence positivity) as an indicator and then collected.

Culture conditions for the cell fusion include, for example, Stemfit™ (AJINOMOTO CO., INC., Japan) as the medium, culture dish coating with 0.5 µg/mL iMatrix™-511 (Nippi, Japan) as the substrate for culture, 37°C as the culture temperature, and 24 hours as the culture period.

The human iPS cells prepared by the method of the present invention has no fluctuation in the fluorescence positive rate even if subculture is repeatedly carried out (for example, 0PDL to 20PDL; where 'PDL' is a population-doubling level.), thereby verifying the stable carriage of an artificial chromosome in the human iPS cells (see Examples described later).

### 2. Method for expressing exogenous gene in exogenous chromosome

The present invention further provides a method for expressing at least one exogenous gene in exogenous chromosome in the human induced pluripotent stem cells prepared in the above 1, or in undifferentiated or differentiated cells derived from the human induced pluripotent stem cells.

According to another aspect of the present invention, the above method includes a method for expressing an exogenous gene in human iPS cells, or in undifferentiated or differentiated cells derived from human iPS cells, the method comprising a step of preparing human iPS cells that comprise an exogenous chromosome comprising an exogenous gene by the method described in section 1 above (hereinafter referred to as "first step"), and a step of expressing the exogenous gene in the human iPS cells, or in the undifferentiated or differentiated cells derived from the human iPS cells induced to differentiate from the human iPS cells (hereinafter referred to as "second step").

Hereinafter, the first step and the second step are described.

### [First step]

In this step, human iPS cells are prepared by the method described in section 1 above. Accordingly, the method described in section 1 above is cited herein as it is.

### [Second step]

This step includes the expression of the exogenous gene in the human iPS cells prepared by the method described in section 1 above, or in the undifferentiated or differentiated cells derived from human iPS cells induced to differentiate from the above-mentioned human iPS cells.

The undifferentiated or differentiated cells derived from the human iPS cells include, but are not limited to, for example, stem cells, hematopoietic stem cells, mesenchymal stem cells, muscle satellite cells (or stem cells of skeletal muscle), progenitor cells, mature cells, or cell population thereof. Examples of such cells include, but are not limited to, cells selected from the group consisting of nerve cells (or neurons), myocardial cells, skeletal muscle cells, smooth muscle cells, T cells, B cells, NK cells, megakaryocytes, hepatocytes, epithelial cells, endothelial cells, pancreatic cells, nephrocytes, and small intestinal cells, or cell populations thereof.

For the preparation of the undifferentiated or differentiated cells derived from the human iPS cells that are induced to differentiate from the human iPS cells, a referral may be made to known methods, such as differentiation induction methods described in: intermediate mesoderm cells (JP Patent Publication (Kohyo) No. 2013-530680 A); hepatocytes (JP Patent Publication (Kokai) No. 2014-128210 A, JP Patent Re-publication (Saihyo) No. 2016/148216); blood cells (JP Patent Re-publication (Saihyo) No. 2014/200030); hematopoietic cells (JP Patent Re-publication (Saihyo) No. 2017/038958); hematopoietic stem cells (N. Suzuki et al., Molecular Therapy, 2013; 21(7): 1424-1431); T cells (JP Patent Re-publication (Saihyo) No. 2016/010148, JP Patent Re-publication (Saihyo) No. 2017/179720); corneal endothelial cells (JP Patent Re-publication (Saihyo) No. 2013/051722); retinal pigment epithelial cells (JP Patent Re-publication (Saihyo) No. 2015/053375); myocardial cells (JP Patent Re-publication (Saihyo) No. 2015/182765); skeletal muscle progenitor cells (JP Patent Re-publication (Saihyo) No. 2016/108288); renal progenitor cells (JP Patent Re-publication (Saihyo) No. 2017/043666); pancreatic progenitor cells (JP Patent Re-publication (Saihyo) No. 2017/188378); neural stem cells (JP Patent Re-publication (Saihyo) No. 2014/069431), nervous system cells (JP Patent Publication (Kokai) No. 2016/131543 A); motor neuron (JP Patent Re-publication (Saihyo) No. 2017/209290); peripheral nerve cells (JP Patent Re-publication (Saihyo) No. 2018/074567); keratinocytes (I. Kogut et al., Methods Mol Biol. 2014; 1195:1-12); and vascular smooth muscle cells (S. Ayoubi et al., Cardiovascular Research, 2017; 113(11):1282-1293).

The above exogenous gene (which may be the DNA of interest described in section 1 above and includes a gene locus or chromosome fragment comprising an exogenous gene) includes, as described above, useful genes such as disease causative genes, therapeutic genes, chromosome fragments comprising these genes, and genes required for cell differentiation. The useful gene includes, but is not limited thereto, for example, genes or DNA encoding proteins: for example, cytokines such as interferon, interleukin, chemokine, granulocyte-colony stimulating factors, tumor necrosis factors, growth factors (for example, platelet-derived growth factors, vascular endothelial growth factors, hepatocyte growth factors, keratinocyte growth factors, and nerve growth factors), nutritional factors (for example, neurotrophic factors and brain-derived neurotrophic factors), erythropoietin, blood-clotting proteins, platelet production promoters, hormones, antibodies (for example, monoclonal antibody and recombinant antibody such as scFV), and enzymes. The useful genes further include therapeutic genes or DNA relating to diseases such as muscular dystrophy, hemophilia, neurodegenerative diseases, autoimmune diseases, allergic diseases, genetic diseases, and tumors and immune system genes or DNA such as T cell receptor (TCR) and human leukocyte antigen (HLA).

An insulator-like DNA sequence may be comprised upstream and/or downstream of a DNA comprising the exogenous gene. The insulator and examples thereof are as described in section 1 above. The insulator is preferably inserted between an enhancer and a promoter. The term "insulator-like" as used herein is used in a broad sense of having an action to block impacts of adjacent chromosomal environment and including an insulator sequence or a sequence analogous thereto.

Further, regulatory sequences, such as an exogenous promoter, enhancer, ribosome binding site, transcription initiation site, and stop sequence, may be comprised as elements required for expressing DNA comprising the exogenous gene, where needed.

Furthermore, the above method may include a step of collecting cells or cell populations comprising the expressed exogenous gene.

The collected cells or cell populations of human iPS cells may be used to prepare differentiated cells, and the undifferentiated or differentiated cells derived from the human iPS cells can be used for regenerative medicine or for new drug development and toxicity assessment. The undifferentiated or differentiated cells derived from the human iPS cells used for new drug development may be derived from, for example, iPS cells induced from somatic cells of patients with an intractable disease such as a genetic disease.

### EXAMPLES

The present invention will be further specifically described in reference to the following Examples, but it is intended that the scope of the invention is not limited to these Examples.

### [Example 1] Transfer of artificial chromosome vector into human iPS cell

### [A] A MAC vector labeled with a drug resistant gene is transferred into human iPS cells thereby to establish an artificial chromosome-carrying human cell line.

### [A.1.1] Microcell fusion and isolation of drug resistant clones

MAC vector (MAC5 or MAC6 (Fig. 1))-comprising CHO cells were seeded in a single culture dish with 10-cm diameter and then cultured to 90% confluency. Into the cells were transferred 12 µg anti-CD9-ScFv fusion MV-H expression plasmid vector and 12 µg MV-F expression plasmid using Lipofectamine 2000 in accordance with the attached protocol. From the day after passaging in three 25 cm²-flasks 24 hours later, colcemid treatment (F12/20% FBS/0.1 µg/mL colcemid) was carried out for 48 hours, medium was exchanged again, and the colcemid treatment (F12/20% FBS/0.1 µg/mL colcemid) was carried out for 24 hours to form micronuclei. After the colcemid treatments, the medium in the flasks was removed and the flasks were filled with cytochalasin B up to 90%. The flask was set in a centrifuge tube to which warm water (34°C) was added to a level at which the flask was not hidden and then centrifuged at 8,000 rpm for 1 hour at 34°C in JLA-10,500 Rotor (BECKMAN). After completion of centrifugal separation, cytochalasin B was collected and removed, and pellets in each flask were collected into a 50-mL tube using 2 mL of serum-free DMEM medium. The pellets were filtered in the order of filter pore sizes 8 µm, 5 µm, and 3 µm and subsequently centrifuged at 2,000 rpm for 10 minutes at room temperature (RT). After centrifugation, the supernatant was removed, pellets in each tube were suspended in a medium for receiving (also referred to as "recipient") cell culture and added to a dish in which the recipient cells have been cultured, and then incubated. The recipient cells were seeded in a single culture dish with 10-cm diameter and coated with 0.5 µg/mL laminin-511 in advance to the human iPS cells, and the cells were cultured to 90% confluency were used. 24 hours later, the cells were re-seeded in 3 culture dishes with 10-cm diameter. Further 24 hours later, selective culture was carried out using 90 µg/mL G418. The selective culture was carried out for 3 to 4 weeks. As a result, 3 drug resistant colonies were obtained from the MAC5-transferred population, while one (1) colony from the MAC6-transferred population (Fig. 2). These were isolated and proliferated, and analyzed as described below.

### [A. 1.2] PCR

Using genomic DNA of the G418 resistant cell line as a template, PCR was carried out using the following primers in order to screen recombinants, and the presence of MAC vectors were confirmed. The sequences of the primers are shown below.
TRANS L1: 5'-TGGAGGCCATAAACAAGAAGAC-3' (SEQ ID NO: 1)
Neo R: 5'-CCCCTTGACCCAGAAATTCCA-3' (SEQ ID NO: 2)

For PCR, GeneAmp 9600 produced by Perkin-Elmer, Inc. as a thermal cycler and EX Taq (TaKaRa) as a Taq polymerase were used, and attached buffer and dNTPs (dATP, dCTP, dGTP, and dTTP) were used in accordance with the recommended conditions. For temperature and cycle conditions, the heat denaturation at 94°C for 1 minute was followed by 35 cycles at 98°C for 10 seconds, 60°C for 30 seconds, and 72°C for 2 minutes. As a result of PCR, all drug resistant clones were positive for MAC vector (Fig. 3).

### [A. 1.3] Karyotype analysis by quinacrine-Hoechst double staining

The positive clones determined by the above-mentioned PCR analysis were subjected to the quinacrine-Hoechst double staining in the same manner as in the method described above. The chromosome images of the clones subjected to the quinacrine-Hoechst double staining were observed under fluorescence microscope (Fig. 4). Two clones examined showed that the MAC vector was carried in a percentage of 65% or more (Fig. 5).

### [A.1.4] Pluripotency analysis using teratoma formation

For the 201B7/MAC5#2 and 201B7/MAC6#1 obtained above, 1 × 10⁶ cells were transplanted to the testicles of SCID mice. Thereafter, teratomas formed 4 to 8 weeks later were collected and fixed with 10% neutral formalin. Then, paraffin-embedded blocks were prepared using Tissue-Tek VIP6AI (Sakura Finetek Japan Co., Ltd.). The paraffin-embedded blocks were thinly sliced to give thinly-sliced sections and then subjected to hematoxylin and eosin-staining for histological analysis. As a result, endodermal tissue (Fig. 6, left panel), mesodermal tissue (Fig. 6, center panel), and ectodermal tissue (Fig. 6, right panel) were observed. These results showed that the MAC vector-transferred human iPS cell lines have pluripotency.

### [B] Stability evaluation of artificial chromosome vector in artificial chromosome-comprising iPS cell

The obtained MAC vector-comprising iPS cells were evaluated for the stability of the artificial chromosome vectors in the human iPS cells by GFP positive rate analysis using a fluorescence microscope and by an artificial chromosome vector content rate using karyotype analysis by quinacrine-Hoechst double staining.

### [B.1.1] Stability evaluation of MAC vector in MAC vector-comprising human iPS cell using fluorescence microscope

A percentage of the GFP positive cells, which show MAC6 carrying cells, of MAC6-comprising 201B7 under neomycin selective culture at 0PDL of starting culture or at 20PDL after long-term culture was measured using a fluorescence microscope and it was consequently found that 90% or more of the cells showed GFP positivity at both 0PDL and 20PDL, which thus means the MAC vector-carrying rates of 90% or more, thereby showing that the MAC vectors in the human iPS cells are stably carried and the gene expression is also stable (Fig. 7).

### [B.1.2] Evaluation of artificial chromosome vector content rate in MAC vector-comprising human iPS cell using karyotype analysis by quinacrine-Hoechst double staining

Using the method described above, the karyotype analysis by quinacrine-Hoechst double staining was carried out to verify the MAC6 carrying cells of MAC6-comprising 201B7 under neomycin derivative G418 nonselective culture at 0PDL of starting culture or at 20PDL after long-term culture, whereby a MAC vector content rate was calculated. At OPDL, the majority of the cells showed the karyotypes of 47, XX, and +MAC6, and 90% of the cells comprised MAC6 (Fig. 8, left panel). At 20PDL of culture in the presence of G418, the majority of the cells similarly showed the karyotypes of 47, XX, and +MAC6, and 90% of the cells comprised MAC6 (Fig. 8, center panel). At 20PDL of culture in the absence of G418, the majority of the cells showed the karyotypes of 47, XX, and +MAC6, and 75% of the cells comprised MAC6 (Fig. 8, right panel). The artificial chromosome vector-comprising human iPS cells were verified to have stable karyotypes even after the long-term culture. Additionally, the artificial chromosome vectors were verified to be maintained for a long term in human iPS cells.

### [Example 2] Transfer of an artificial chromosome vector into human iPS cell derived from patient with Duchenne muscular dystrophy (DMD)

### [A] A DYS-HAC2 vector labeled with a drug resistant gene is transferred into an iPS cell line derived from a human DMD patient.

### [A.1.1] Microcell fusion and isolation of drug resistant clone

DYS-HAC2 vector-comprising CHO cells were seeded in a single culture dish with 10-cm diameter and then cultured to 90% confluency. Into the cells were transferred 12 µg wild-type MV-H expression plasmid vector or an anti-CD71-ScFv or CD9-ScFv fusion MV-H expression plasmid vector and 12 µg MV-F expression plasmid using Lipofectamine 2000 in accordance with the attached protocol. From the day after passaging in three 25 cm²-flasks 24 hours later, colcemid treatment (F12/20% FBS/0.1 µg/mL colcemid) was carried out for 48 hours, medium was exchanged again, and the colcemid treatment (F12/20% FBS/0.1 µg/mL colcemid) was carried out for 24 hours to form micronuclei. After the colcemid treatments, the medium in the flasks was removed and the flasks were filled with cytochalasin B up to 90%. The flask was set in a centrifuge tube to which warm water (34°C) was added to a level at which the flask was not hidden and centrifuged at 8,000 rpm for 1 hour at 34°C in JLA-10,500 Rotor (BECKMAN). After completion of centrifugal separation, cytochalasin B was collected and removed, and pellets in each flask were collected into a 50-mL tube using 2 mL serum-free DMEM medium. The pellets were filtered in the order of filter pore sizes 8 µm, 5 µm, and 3 µm and subsequently centrifuged at 2,000 rpm for 10 minutes at room temperature (RT). After centrifugation, the supernatant was removed, pellets in each tube were suspended in medium for recipient cell culture and then added to a dish in which the recipient cells have been cultured, and incubated. The recipient cells were seeded in a single culture dish with 10-cm diameter and coated with Geltrex™ LDEV-Free hESC-qualified (Thermo Fisher Scientific) (5% concentration) in advance to the human iPS cells (DMD-iPS#1) and those cultured to 90% confluency were used. 24 hours later, the cells were re-seeded in 3 culture dishes with 10-cm diameter. Further 24 hours later, selective culture was carried out using 90 µg/mL G418. The selective culture was carried out for 3-4 weeks. As a result, 10 drug-resistant colonies from the DYS-HAC2-transfer group which was obtained using the wild-type MV-H, 12 drug-resistant colonies from the DYS-HAC2-transfer group which was obtained using the anti-CD71-ScFv fusion MV-H expression plasmid vector, and 10 drug-resistant colonies from DYS-HAC2-transfer group which was obtained using the CD9-ScFv fusion MV-H expression plasmid vector, were obtained in the total of 32 clones. These were isolated and proliferated, and analyzed as follows.

### [A.1.2] PCR

Using genomic DNA of the 32 G418-resistant clones obtained above as a template, PCR was carried out using the following primers to screen recombinants. The sequences of the primers are shown below.
NeoF: 5'-CACAACAGACAATCGGCTGCTCT-3' (SEQ ID NO: 3)
DloxP3L: 5'-GCATGGGGGAGGAGAGAAGAGAGATGTA-3' (SEQ ID NO: 4)
hCMV586: 5'-CGTAACAACTCCGCCCCATT-3' (SEQ ID NO: 5)

For PCR, GeneAmp™ 9600 (manufactured by Perkin-Elmer) as a thermal cycler and EX Taq (TaKaRa) as a Taq polymerase were used, and attached buffer and dNTPs (dATP, dCTP, dGTP, and dTTP) were used in accordance with the recommended conditions. For temperature and cycle conditions, the heat denaturation at 94°C for 1 minute was followed by 35 cycles at 98°C for 10 seconds, 60°C for 30 seconds, and 72°C for 2 minutes. As the result of PCR, 11 clones out of the analyzed 32 clones were positive (Fig. 9).

### [A.1.3] Screening of drug resistant clone by FISH analysis

The clones obtained above were FISH-analyzed by the method described by Shinohara et al. (Human Molecular Genetics, 10:1163-1175, 2001). Fig. 10 shows the FISH staining result of the clone in which DYS-HAC2 was transferred into the human iPS cell line (DMD-iPS#1) when human alpha satellite and RP11-954B16 were used as the probes. Human chromosome 13, human chromosome 21, or DYS-HAC2 is stained with red color (arrow). The dystrophin gene is stained with green color (arrowhead). In Fig. 10, an enlarged DYS-HAC2 vector is shown in a small window.

### [A.1.4] Pluripotency analysis by teratoma formation

For the DMD-iPS DYS-HAC2 #8 obtained above, 1 × 10⁶ cells were transplanted to the testicles of SCID mice. Thereafter, teratomas formed 4-8 weeks later were collected and fixed with 10% neutral formalin. Then, paraffin-embedded blocks were prepared using Tissue-Tek VIP6AI (Sakura Finetek Japan Co., Ltd.). The paraffin-embedded blocks were thinly sliced to give thinly-sliced sections and then hematoxylin and eosin-stained for histological analysis. As a result, endodermal tissue (Fig. 11, left panel), mesodermal tissue (Fig. 11, center panel), and ectodermal tissue (Fig. 11, right panel) were observed. These results show that the DYS-HAC2 vector-comprising iPS cell lines derived from a human DMD patient have pluripotency.

### [Example 3] Transfer of human chromosome 21 fragment into human iPS cell

### [A] A human chromosome 21 fragment labeled with a drug resistant gene is transferred into human iPS cells thereby to establish a human chromosome 21 fragment-carrying human cell line (iPS cell line as a Down Syndrome model).

### [A.1.1] Microcell fusion and isolation of drug resistant clone

Human chromosome 21 fragment-comprising CHO cells were seeded in a single culture dish with 10-cm diameter and then cultured to 90% confluency. Into the cells were transferred 12 µg anti-CD9-ScFv fusion MV-H expression plasmid vector and 12 µg MV-F expression plasmid using Lipofectamine 2000 in accordance with the attached protocol. From the day after passaging to three 25 cm²-flasks 24 hours later, colcemid treatment (F12/20% FBS/0.1 µg/mL colcemid) was carried out for 48 hours, medium was exchanged again, and the colcemid treatment (F12/20% FBS/0.1 µg/mL colcemid) was carried out for 24 hours to form micronuclei. After the colcemid treatments, the medium in the flasks was removed, and the flasks were filled with cytochalasin B up to 90%. The flask was set in a centrifuge tube to which warm water (34°C) was added to a level at which the flask was not hidden and centrifuged at 8,000 rpm for 1 hour at 34°C in JLA-10,500 Rotor (BECKMAN). After centrifugation, cytochalasin B was collected and removed, and pellets in each flask were collected into a 50-mL tube using 2 mL serum-free DMEM medium. The pellets were filtered in the order of filter pore sizes 8 µm, 5 µm, and 3 µm and subsequently centrifuged at 2,000 rpm for 10 minutes at room temperature (RT). After centrifugation, the supernatant was removed, pellets in each tube were suspended in a medium for recipient cell culture, and then added to a dish in which the recipient cells have been cultured, and incubated. The recipient cells were seeded in a single culture dish with 10-cm diameter coated with Geltrrex™ LDEV-Free hESC-qualified (5% concentration) in advance to the human iPS cells, and those cultured to 90% confluency were used. 24 hours later, the cells were re-seeded in 3 culture dishes with 10-cm diameter. Further 24 hours later, selective culture was carried out using 50 µg/mL G418. The selective culture was carried out for 3-4 weeks. As a result, one (1) drug resistant colony was obtained. This colony was isolated and proliferated.

### [A.1.2] Karyotype analysis by quinacrine-Hoechst double staining

The above drug-resistant clone was subjected to the quinacrine-Hoechst double staining in the same manner as in the method described above and confirmed that a triplet of chromosome 21 was independently comprised by the transfer of chromosome 21 (Fig. 12).

### [A.1.3] Pluripotency analysis by teratoma formation

For the exogenous human No. 21 fragment-comprising human iPS cells obtained above, 1 × 10⁶ cells were transplanted to testicles of SCID mice. Thereafter, teratomas formed 4 to 8 weeks later were collected and fixed with 10% neutral formalin. Then, paraffin-embedded blocks were prepared using Tissue-Tek VIP6AI (Sakura Finetek Japan Co., Ltd.). The paraffin-embedded blocks were thinly sliced to give thinly-sliced sections and hematoxylin and eosin-stained to carry out histological analysis. As a result, endodermal tissue (Fig. 13, left panel), mesodermal tissue (Fig. 13, center panel), and ectodermal tissue (Fig. 13, right panel) were observed. These results showed that the exogenous human No. 21 fragment-comprising human iPS cells have pluripotency.

### [Example 4] Gene transfer into a MAC vector in MAC vector-comprising human iPS cells

### [A] Three types of plasmid vectors were transferred into a MAC vector in human iPS cells by a 3-gene co-transfer method in order to verify the gene transfer to an artificial chromosome in human iPS cells.

### [A.1.1] Preparation of plasmid vectors for inserting genes into a MAC vector in human iPS cells

Cassette vectors were prepared as follows.

### (1) pBG2-V0binsElucins

An AscI/AvrII fragment of CAG-Eluc vector was inserted into an AscI/NheI cleavage site of pBG2-V0 into an AscI/AvrII site of pBG2-V0b (1C).

### (2) GLV2-EF1a-tdTomato

EcoRI-XbaI-EcoRI was inserted into an EcoRI cleavage site of tDNA pEF BGHpA. An NheI/AvrII cleavage fragment of pCMV tdTomato was inserted into the XbaI site of tDNA pEF BGHpA-XbaI (tDNA EFla tdTomato). An AscI/AvrII cleavage fragment of PCR product at BxbI-PhiC31_CL_F/BxbI-PhiC31_CL_R of pBG2-Vla (2A) was inserted into AscI/NheI of tDNA Efia tdTomato (GLV2-EF1a-tdTomato).

### (3) GLV3-Neo-BFP

An EcoRI cleavage fragment of PCR product at BFP_cl_EcoRI_FBFP_cl_EcoRI_R of pTagBFP2 was inserted into the EcoRI cleavage site of tDNA-EF1aBGHpA (tDNA-EF1a-BFP). The AscI/AvrII cleavage fragment of PCR product at PhiC31att_3'HPRT_Asc1_F/PhiC31att_3'HPRT_cl R of pBG2-V2a (3A) was inserted into the AscI/NheI cleavage site of tDNA-EF1a-BFP (GLV3-EF1a-BFP). An AvrII/AgeI fragment of PCR product at SA-Neo AvrII Fw/SA-Neo Agel Rv closer to pBG2-v1b1, an AscI/AvrII fragment of PCR product at PhiC31 AscI F/PhiC31 AvrII R closer to GLV3-EF1a-BFP, and a fragment of AgeI/HindIII Oligo were inserted into an AscI/HindIII fragment of GLV3-EFla-BFP (GLV3-Neo-BFP).

### [A.1.2] Transfection and isolation of G418 resistant clones

The gene transfer was carried out using an electroporator NEPA21 (NEPAGENE Co. Ltd.). 3 × 10⁶ iPS cells were washed with 5 mL of PBS and centrifuged at 1200 rpm at room temperature for 5 minutes and the cells were collected. The centrifugal separation was further carried out using 5 mL of Opti-MEM (Thermo Fisher Scientific Inc.) at 1200 rpm at room temperature for 5 minutes and the cells were collected. The cells were suspended in 90 µL of Opti-MEM. With this, 3.5 µg of pBG2-V0binsElucins, 7 µg of GLV2-EF1a-tdTomato, 10.5 µg of GLV3-EF1a-BFP, 3 µg of a Cre expression vector, 3 µg of a Bxb1 integrase expression vector, and 3 µg of PhiC31 integrase expression vector were mixed, and the mixture was transferred using a NEPA21 electroporator in accordance with the attached protocol under the conditions of Poring Pulse (Voltage: 135 V, Pulse Length: 5.0 msec, Pulse Interval: 50 msec, Number of Pulses: 2, Decay Rate: 10%, Polarity: +) and Transfer Pulse (Voltage: 20 V, Pulse Length: 50.0 msec, Pulse interval: 50.0 msec, Number of Pulses: 5, Decay Rate: 40%, Polarity: +/-). From 48 hours after the transfer, the cells were cultured for 3-4 weeks under G418 (90 µg/mL) selective culture and, as a result, fluorescence positive drug resistant colonies appeared. One (1) colony obtained by a single transfer from the MAC vector-comprising human iPS cells was isolated and proliferated, and analyzed as follows.

### [B] Screening of drug resistant clones

### [B.1.1] PCR

Using genome DNA of the HAT resistant cell line as a template, PCR was carried out using the following primers to screen recombinants and confirm whether the insertion of site-specifically transferred genes occurred. The primer sequences are shown below.
TRANS L1: 5'-TGGAGGCCATAAACAAGAAGAC-3' (SEQ ID NO: 6)
SIM Neo Rv: 5'-CGCCTTGAGCCTGGCGAACA-3' (SEQ ID NO: 7)
tdTomatoF: 5'-ACAACAACATGGCCGTCATC-3' (SEQ ID NO: 8)
tdTomatoR: 5'-CATGCCGTACAGGAACAGGT-3' (SEQ ID NO: 9)
ElucF: 5'-CGATCCTGGTCTTCACCACT-3'(SEQ ID NO: 10)
ElucR: 5'-TGCAGAGGCTTGAATGTTTG-3' (SEQ ID NO: 11)
BFP Fw: 5'-CTGGAAGGCAGAAACGACAT-3' (SEQ ID NO: 12)
BFP Rv: 5'-TGCTAGGGAGGTCGCAGTAT-3' (SEQ ID NO: 13)

For PCR, GeneAmp 9600 produced by Perkin-Elmer, Inc. as a thermal cycler and LA Taq (TaKaRa) as a Taq polymerase were used, and attached buffer and dNTPs (dATP, dCTP, DGTP, and dTTP) were used in accordance with the recommended conditions. For temperature and cycle conditions, the heat denaturation at 94°C for 1 minute was followed by 35 cycles at 98°C for 10 seconds, 60°C for 30 seconds, and 72°C for 2 minutes. As a result of PCR, 1 clone was positive (Fig. 14).

### [B.1.2] Analysis of fluorescence positive frequency by cell analyzer

Positive frequency of EGFP, BFP, and tdTomato were measured using BD Fortessa to quantify fluorescence positive frequency of each clone. Each clone was suspended at 1 × 10⁵ cells in 500 µL of PBS (-) and then measured. As a result, the cells were clones comprising triple positive cells of EGFP, BFP, and tdTomato.

### [B.1.3] Luminescence measurement

Luminescence measurement was carried out to detect emerald luciferase expression of each clone in accordance with the standard procedure of a reagent using a luciferase assay system produced by Toyo B-Net Co., Ltd. 1 × 10⁴ cells were used to carry out the measurement and emerald luciferase expression was positive.

### [B.1.4] Screening of drug resistant clones by FISH analysis

In the clones obtained above, a mouse cot-1 sequence was used as a red color probe by the method described in the report by Shinohara et al. (Human Molecular Genetics, 10:1163-1175, 2001) (the arrow in Fig. 15). Fish analysis was carried out using pBG2-V0binsElucins, GLV2-EF1a-tdTomato, or GLV3-EFla-BFP as a green color probe (the arrowhead in Fig. 15). As a representative example, pBG2-V0binsElucins showing carrying the emerald luciferase gene (Eluc) and the staining result of the mouse cot-1 sequence used as the probe are shown. The results showed that the MAC vector into which the exogenous DNA was inserted was carried while being independent from the host chromosome.

### [B.1.5] Analysis of pluripotency by teratoma formation

For the exogenous DNA-carrying MAC vector-comprising human iPS cells obtained above, 1 × 10⁶ cells were transplanted to testicles of SCID mice. Thereafter, teratomas formed 4 to 8 weeks later were collected and then fixed with 10% neutral formalin. Subsequently, paraffin-embedded blocks were prepared using Tissue-Tek VIP6AI (Sakura Finetek Japan Co., Ltd., Japan). The paraffin-embedded blocks were thinly sliced to give thinly-sliced sections and hematoxylin and eosin-stained to carry out histological analysis. As a result, endodermal tissue (Fig. 16, left panel), mesodermal tissue (Fig. 16, center panel), and ectodermal tissue (Fig. 16, right panel) were observed. These results showed that the exogenous DNA-carrying MAC vector-comprising human iPS cells have pluripotency.

### [C] MAC vector stability and gene expression persistency in the exogenous DNA-loaded MAC vector-comprising human iPS cells

The obtained exogenous DNA-carrying MAC vector-comprising iPS cells were evaluated for the stability of an artificial chromosome vector in the human iPS cells by the analysis of fluorescence protein positive rate using a fluorescence microscope and the analysis of artificial chromosome vector content rate using the karyotype analysis by quinacrine-Hoechst double staining.

### [C.1.1] Evaluation on MAC vector stability and gene expression persistency in the exogenous DNA-carrying MAC vector-comprising human iPS cells using fluorescence microscope

The above obtained exogenous DNA-carrying MAC vector-comprising human iPS cells were evaluated for GFP positive rate, tdTomato positive rate, and BFP2 positive rate of the exogenous DNA-carrying MAC vector-comprising human iPS cells using a fluorescence microscope at 0PDL of starting culture and at 20PDL of long-term culture under neomycin nonselective culture. As a result of the measurement, some cells with all GFP, tdTomato, and BFP2 being positive at 0PDL were present (Fig. 17). Gene expression was recognized even after the long-term culture 20PDL. These showed that the exogenous DNA-carrying MAC vector can express the exogenous DNA for a long term in human iPS cells.

### [C.1.2]

Evaluation of the rate of artificial chromosome vector contained in the exogenous DNA-carrying MAC vector-comprising human iPS cells by karyotype analysis using quinacrine-Hoechst double staining

Using the method described above, the karyotype analysis using quinacrine-Hoechst double staining was carried out to verify the MAC6 carrying rate in exogenous DNA-carrying MAC vector-comprising 201B7 under neomycin derivative G418 nonselective culture at 0PDL of starting culture or at 20PDL after long-term culture, whereby the MAC vector content rate was calculated. At OPDL, the majority of the cells showed the karyotypes of 47, XX, and +MAC6, and 95% of the cells comprised MAC6 (Fig. 18, left panel). At 20PDL of culture in the presence of G418, the majority of the cells similarly showed the karyotypes of 47, XX, and +MAC6 and 90% of the cells comprised MAC6 (Fig. 18, center panel). At 20PDL of culture in the absence of G418, the majority of the cells showed the karyotypes of 47, XX, and +MAC6 and 75% of the cells comprised MAC6 (Fig. 18, right panel). The artificial chromosome vector-comprising human iPS cells were verified to have stable karyotypes even after the long-term culture. Additionally, the exogenous DNA-acrrying artificial chromosome vector was verified to be maintained for a long term in human iPS cells.

### INDUSTRIAL APPLICABILITY

The method of the present invention enables the production of human iPS cells comprising an artificial chromosome of a megabase (Mb) size having a DNA of interest and the expression of a DNA of interest (for example, an exogenous gene) in human iPS cells, or undifferentiated or differentiated cells derived from the human iPS cells induced to differentiate from the human iPS cells. This method is useful for regenerative medicine which utilizes human iPS cells and for new drug development.

### SEQUENCE LISTING FREE TEXT

### SEQ ID NOs: 1 to 13: primers

All publications, patents, and patent applications cited herein are incorporated herein by reference in their entirety.

## Claims

1. A method for producing human induced pluripotent stem (iPS) cells comprising an exogenous chromosome, comprising the following steps of:
providing a donor cell expressing a viral envelope protein on the cell surface and comprising an exogenous chromosome having a DNA of interest;
preparing microcells from the donor cell; and
co-culturing and fusing the microcells and human iPS cells as a recipient cell in the absence of feeder cells using an MMCT (Microcell-Mediated Chromosome Transfer) method, thereby introducing the exogenous chromosome having a DNA of interest into the human iPS cells.

2. The method according to claim 1, wherein the exogenous chromosome is an artificial chromosome or a mammalian artificial chromosome.

3. The method according to claim 1 or 2, wherein the viral envelope protein is a measles virus-derived envelope protein or a modified protein thereof.

4. The method according to claim 3, wherein the modified protein is a fusion protein of a measles virus-derived H protein with an anti-CD9 antibody, anti-CD 13 antibody or anti-CD71 antibody, or with an ScFv of the antibody.

5. The method according to any one of claims 1 to 4, wherein the donor cell is a mammalian cell.

6. The method according to claim 5, wherein the mammalian cell is a rodent cell.

7. The method according to any one of claims 1 to 6, wherein the DNA of interest is an exogenous gene, a locus, or a chromosome fragment.

8. A method for expressing an exogenous gene in human iPS cells or in undifferentiated or differentiated cells derived from the human iPS cells, the method comprising the following steps of:
preparing human iPS cells comprising an exogenous chromosome having an exogenous gene by the method according to any one of claims 1 to 7; and
expressing the exogenous gene in the human iPS cells or in the undifferentiated or differentiated cells derived from the human iPS cells and induced to differentiate from the human iPS cells.

9. The method according to claim 8, wherein the undifferentiated or differentiated cell derived from the human iPS cells is a stem cell, a hematopoietic stem cell, a mesenchymal stem cell, a muscle satellite cell, a progenitor cell, a mature cell, or a cell population thereof.

10. The method according to claim 9, wherein the undifferentiated or differentiated cell derived from the human iPS cells is selected from the group consisting of nerve cells, myocardial cells, skeletal muscle cells, smooth muscle cells, T cells, B cells, NK cells, megakaryocytes, hepatocytes, epithelial cells, endothelial cells, pancreatic cells, nephrocytes, and small intestinal cells.

11. The method according to any one of claims 8 to 10, comprising an insulator-like DNA sequence upstream and/or downstream of a DNA comprising the exogenous gene.


SEQUENCE LISTING

<110>  NATIONAL UNIVERSITY CORPORATION TOTTORI UNIVERSITY
       Trans Chromosomics, Inc.

<120>  Method of Producing Human Induced Pluripotent Stem Cells
       Comprising Exogenous Chromosome

<130>  PH-8044-PCT

<150>  JP 2018-191894
<151>  2018-10-10

<160>  13

<170>  PatentIn version 3.5

<210>  1
<211>  22
<212>  DNA
<213>  Artificial

<220>
<223>  primer

<400>  1
tggaggccat aaacaagaag ac                                                22


<210>  2
<211>  21
<212>  DNA
<213>  Artificial

<220>
<223>  primer

<400>  2
ccccttgacc cagaaattcc a                                                 21


<210>  3
<211>  23
<212>  DNA
<213>  Artificial

<220>
<223>  primer

<400>  3
cacaacagac aatcggctgc tct                                               23


<210>  4
<211>  28
<212>  DNA
<213>  Artificial

<220>
<223>  primer

<400>  4
gcatggggga ggagagaaga gagatgta                                          28

<210>  5
<211>  20
<212>  DNA
<213>  Artificial

<220>
<223>  primer

<400>  5
cgtaacaact ccgccccatt                                                    20


<210>  6
<211>  22
<212>  DNA
<213>  Artificial

<220>
<223>  primer

<400>  6
tggaggccat aaacaagaag ac                                                 22


<210>  7
<211>  20
<212>  DNA
<213>  Artificial

<220>
<223>  primer

<400>  7
cgccttgagc ctggcgaaca                                                    20


<210>  8
<211>  20
<212>  DNA
<213>  Artificial

<220>
<223>  primer

<400>  8
acaacaacat ggccgtcatc                                                    20


<210>  9
<211>  20
<212>  DNA
<213>  Artificial

<220>
<223>  primer

<400>  9
catgccgtac aggaacaggt                                                    20


<210>  10

<211>  20
<212>  DNA
<213>  Artificial

<220>
<223>  primer

<400>  10
cgatcctggt cttcaccact                                                    20


<210>  11
<211>  20
<212>  DNA
<213>  Artificial

<220>
<223>  primer

<400>  11
tgcagaggct tgaatgtttg                                                    20


<210>  12
<211>  20
<212>  DNA
<213>  Artificial

<220>
<223>  primer

<400>  12
ctggaaggca gaaacgacat                                                    20


<210>  13
<211>  20
<212>  DNA
<213>  Artificial

<220>
<223>  primer

<400>  13
tgctagggag gtcgcagtat                                                    20